(19) Europäisches Patentamt
European Patent Office
Office européen des brevets

(11) Veröffentlichungsnummer: **0 019 069**
**B1**

(12) # EUROPÄISCHE PATENTSCHRIFT

(45) Veröffentlichungstag der Patentschrift: **25.07.84**

(21) Anmeldenummer: **80101719.5**

(22) Anmeldetag: **01.04.80**

(51) Int. Cl.³: **C 07 C 177/00, C 07 D 307/935, A 61 K 31/557**

(54) **6-Keto-PGE1- und PGI1-Derivate, Verfahren zu ihrer Herstellung und ihre Verwendung als Arzneimittel.**

(30) Priorität: **06.04.79 DE 2913856**

(43) Veröffentlichungstag der Anmeldung: **26.11.80 Patentblatt 80/24**

(45) Bekanntmachung des Hinweises auf die Patenterteilung: **25.07.84 Patentblatt 84/30**

(84) Benannte Vertragsstaaten: **AT BE CH DE FR GB IT LI NL SE**

(56) Entgegenhaltungen:
**BE - A - 870 531**
**FR - A - 2 376 134**

**UNLISTED DRUGS, Band 31, Nr. 4, April 1979, Seite 50 Zusammenfassung Nr. g 6-KPGE**
**ANGEWANDTE CHEMIE, Band 17, Nr. 5, Mai 1978, International Edition, Seiten 360-379 K.C. NICOLAOU et al.: "Synthesis and biological properties of prostaglandin endoperoxides, thromboxanes and prostacyclins"**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Beck, Gerhard, Dr.**
**Gustav-Freytag-Strasse 24**
**D-6000 Frankfurt am Main (DE)**
Erfinder: **Lerch, Ulrich, Dr.**
**Schwalbenweg 19**
**D-6238 Hofheim am Taunus (DE)**
Erfinder: **Schölkens, Bernward, Dr.**
**Am Fliedergarten 1**
**D-6233 Kelkheim (Taunus) (DE)**
Erfinder: **Rupp, Richard Helmut, Dr.**
**Johannesallee 24**
**D-6230 Frankfurt am Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 019 069 B1**

**Beschreibung**

Prostaglandine sind eine Gruppe von Fettsäuren, die in zahlreichen Geweben und Organen von Menschen und Tier vorkommen. Das Grundgerüst der natürlichen Prostaglandine besteht aus 20 Kohlenwasserstoffatomen, die in Form eines Fünfrings und zweier benachbarter linearer Seitenketten angeordnet sind.

Die pharmakologischen Effekte der Prostaglandine erstrecken sich u.a. auf die Gebiete der Reproduktion, des Bronchialmuskeltonus, des Blutdrucks und der Gastroenterologie. Die pharmakologischen Eigenschaften der natürlichen Prostaglandine sind Gegenstand zahlreicher Übersichtsartikel, z.B. N. H. Andersen und P. W. Ramwell in Arch. Internal Med. *133,* 30 (1974); R. L. Jones in Pathobiology Ann. *1972,* 359; J. Pike in Scient. American *225,* 84 (1971) oder M. P. L. Caton in Progress in Med. Chem. vol. 7, ed.: Butterworth, London 1971.

Die Synthese von nicht natürlich vorkommenden Analogen von Prostansäuren, in denen die Vielzahl der pharmakologischen Wirkungen der natürlichen Prostaglandine differenziert sind, gewinnt zunehmend an Bedeutung.

Die vorliegende Erfindung betrifft neue Verbindungen der Formel I

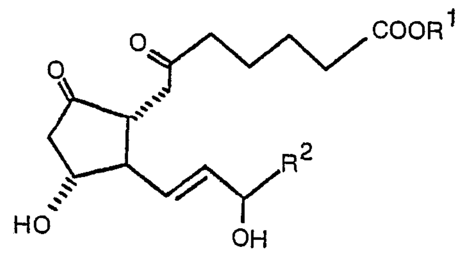

I

die strukturell mit den natürlichen Prostaglandinen verwandt sind und in welcher bedeuten

$R^1$ Wasserstoff oder einen geradkettigen oder verzweigten Alkylrest mit bis zu 8 Kohlenstoffatomen, oder einen geradkettigen oder verzweigten ungesättigten aliphatischen Kohlenwasserstoff-rest mit bis zu 6 Kohlenwasserstoffrest mit 3 bis 7 Kohlenstoffatomen oder einen arali. phatischen Kohlenwasserstoffrest mit 7 bis 9 Kohlenstoffatomen, oder ein physiologisch verträgliches Metall-, $NH_4$— oder Ammoniumion, das sich von einem primären, sekundären oder tertiären Amin ableitet, oder ein Tetraalkylammoniumion,

$R^2$ einen geradkettigen oder verzweigten Alkylrest mit bis zu 8 Kohlenstoffatomen,. der substituiert ist mit $\alpha$- oder $\beta$-Furyl.

Unter den für $R^1$ genannten Substituenten sind die folgenden bevorzugt:

Wasserstoff, ein geradkettiger oder verzweigter Alkylrest mit bis zu 8 C-Atomen, ein geradkettiger oder verzweigter ungesättigter aliphatischer Kohlenwasserstoffrest mit bis zu 4 C-Atomen, ein cycloaliphatischer Kohlenwasserstoffrest mit 5—7 C-Atomen, ein araliphatischer Kohlenwasserstoffrest mit 7 bis 8 C-Atomen.

Insbesondere sind bevorzugt:

Wasserstoff, Methyl, Äthyl, n-Butyl, n-Pentyl, n-Hexyl, n-Heptyl, 2-Propyl, 2-Butyl, 2-Pentyl, 3-Hexyl, 2-Methylpropyl, 2-Methylbutyl, 4,4-Dimethylpentyl, 5,5-Dimethylhexyl, Cyclopentyl, Cyclohexyl, Cycloheptyl.

Under den für $R^2$ genannten Substituenten sind bevorzugt geradkettige oder verzweigte Alkylrest mitbis zu 5 C-Atomen, die substituiert sind mit $\alpha$- oder $\beta$-Furyl, z.B. 3-Furyl-2-äthyl und 2-Furyl-2-äthyl.

Gegenstand der Erfindung ist ferner ein Verfahren zur Herstellung von Verbindungen der Formel I, das dadurch gekennzeichnet ist, daß man

a) aus einer Verbindung der Formel II

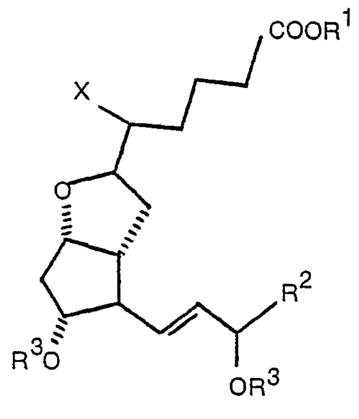

II

worin $R^1$ und $R^2$ die zur Formel I angegebene Bedeutung haben, $R^3$ eine leicht abspaltbare Schutzgruppe und X ein Halogenatom bedeutet, mit Hilfe einer Base HX abspaltet wobei eine Verbindung der Formel III

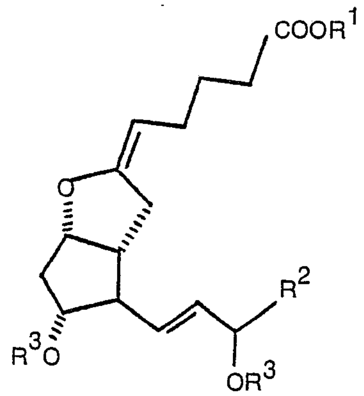

III

erhalten wird,

worin $R^1$ und $R^2$ die zur Formel angegebene Bedeutung haben und $R^3$ eine abspaltbare Schutzgruppe bedeutet,

b) eine Verbindung der Formel III sauer hydrolysiert zu einer Verbindung der Formel IV,

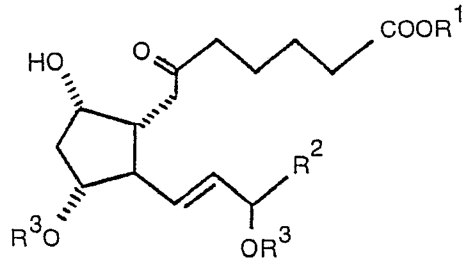

IV

worin $R^1$ und $R^2$ die zur Formel I angegebene Bedeutung haben und $R^3$ eine leicht abspaltbare Schutzgruppe bedeutet,

c) eine Verbindung der Formel V

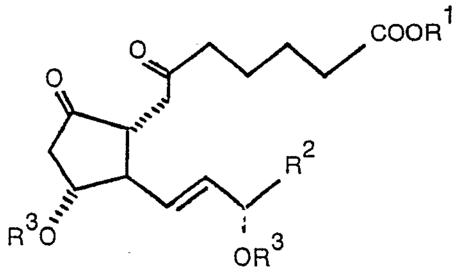

V

worin $R^1$ und $R^2$ die zur Formel I angegebene Bedeutung haben und $R^3$ eine leicht abspaltbare Schutzgruppe bedeutet,

d) einer Verbindung der Formel V die Schutzgruppen $R^3$ unter geeigneten neutralen oder sauren Bedingungen abspaltet, wobei eine Verbindung der Formel I gebildet wird, worin $R^1$ und $R^2$ die zur Formel I angegebene bedeutung haben,

e) gegebenenfalls eine Verbindung der Formel I, in der $R^1$ nicht Wasserstoff oder ein Kation darstellt, durch saure oder alkalische Hydrolyse überführt in eine Verbindung der Formel I, in der $R^1$ Wasserstoff oder ein physiologisch verträgliches Kation bedeutet, und

f) gegebenenfalls in einer Verbindung der Formel I, in der $R^1$ ein physiologisch verträgliches Metall-, $NH_4$- oder Ammoniumion, das sich von einem primären, sekundären oder tertiären Amin ableitet oder ein Tetraalkylammoniumion bedeutet, das Kation $R^1$ gegen ein anderes austauscht.

Für die Herstellung der im erfindungsgemäßen Verfahren als Ausgangsmaterial verwendeten Prostaglandinderivate der Formel II kann man analog dem Verfahren arbeiten, wie es z.B. in der DE—OS 28 11 950 beschrieben ist.

Die Abspaltung von HX, wobei X vorzugsweise Brom oder Jod bedeutet, aus Verbindungen der Formel II unter Bildung von Verbindungen der Formel III verläuft unter der Einwirkung von Basen in Gegenwart oder Abwesenheit eines Lösungsmittels.

Als Basen kommen sowohl anorganische wie organische in Betracht, wie z.B. Alkalimetallhydroxyde oder -carbonate, Alkoholate wie z.B. Natriummethylat oder Kaliumtertiärebutylat, Amine wie z.B. Triäthylamin, 4-Dimethylaminopyridin, Dicyclohexyläthylamin oder 1,4-Diazabicyclo[2,2,2]octan oder Amide wie z.B. 1,5-Diazabicyclo[3,4,0]nonen-5 (DBN) oder 1,5-Diazabicyclo[5,4,0]undecen-5 (BDU). Die Hydrolyse von Verbindungen der Formel III zu Verbindungen der Formel IV wird durch Säurekatalyse bei Temperaturen zwischen —10°C und +20°C zweckmäßigerweise in einem alkoholischen oder wäßrigen organischen Lösungsmittel durchgeführt. Als Säuren eignen sich verdünnte Mineralsäuren oder organische Säuren wie p-Toluolsulfonsäure, Oxalsäure oder Essigsäure.

Die Verbindungen der Formel IV werden durch Oxydation in Verbindungen der Formel V überführt. Als Oxydationsmittel eignen sich insbesondere Chromsäuren, Chromsäure-Pyridin-Komplexe, Jones-Reagenz, Pyridiniumchlorchromat. Als geeignete Lösungsmittel eignen sich solche, die selbst nicht oxydiert werden können, wie z.B. Aceton, Äther, Methylchlorid.

Die Verbindungen der Formel V, worin —$COOR^1$ eine Estergruppe darstellt, werden durch Entfernung der Schutzgruppen $R^3$ in Gegenwart von verdünnter Mineralsäure oder organischer Säuren wie p-Toluol-sulfonsäure, Oxalsäure oder Essigsäure, zweckmäßigerweise in einem wässrig/organischen Lösungsmittel, wie z.B. Tetrahydrofuran/Wasser, in Verbindungen der Formel I, worin —$COOR^1$ eine Estergruppe darstellt, überführt.

Verbindungen der Formel I, worin $R^1$ einen Alkylrest bedeutet, können in üblicher Weise in alkalischem Medium zu Verbindungen der Formel I, worin $R^1$ Wasserstoff oder vorzugsweise ein Kation bedeutet, verseift werden, z.B. mit NaOH oder KOH in einem niedermolekularen Alkohol wie Methanol oder Äther wie Dimethoxyäthan oder THF, gegebenenfalls in Gegenwart von Wasser. Vorteilhafterweise benutzt man die äquimolare Menge oder einen sehr geringen Überschuß an Alkalihydroxyd, so daß man das Alkalisalz der Formel I ($R^1$ = Alkalimetallion) durch Verdampfen des Lösungsmittels erhält.

Das Alkalikation läßt sich an Ionenaustauschern in üblicher Weise gegen beliebige Kationen austauschen. Dazu läßt man die Lösung des Alkalisalzes einer Verbindung der Formel I durch eine mit einem Kationaustauscher wie z.B. Amberlite CG—50 oder Dowex CCR—2 gefüllte Säule laufen.

Der Kationaustauscher ist mit dem erwünschten Kation beladen, z.B. mit einem Ammoniumion, das sich von einem primären, sekundären oder tertiären Amin ableitet. Das erwünschte Salz erhält man durch Eindampfen des Eluats. Die Verbindungen der Formel II, III, IV und V können als Diastereomerengemisch bezüglich der Stellung der Hydroxylgruppe am Kohlenstoffatom 15 (Prostaglandinnomenklatur), als reine — oder $\beta$-Isomere oder in Form von optisch aktiven Antipoden für die nachfolgenden Reaktionen eingesetzt werden. Die Trennung von Stereoisomeren bzw. Antipoden kann aber auch nach jeder folgenden Reaktionsstufe erfolgen. Das bedeutet, daß alle beschriebenen Reaktionen mit Diastereomerengemischen, reinen Diastereomeren oder optisch aktiven Antipoden durchgeführt werden können.

Sofern die einzelnen Reaktionsstufen nicht bereits in genügend reiner Form anfallen, so daß sie für den folgenden Reaktionsschritt eingesetzt werden können, empfiehlt sich eine Reinigung mittels z.B. Säulen-, Dünnschicht- oder Hochdruckflüssigkeitschromatographie.

Die Verbindungen der Formel I zeichnen sich aus durch hemmende Wirkung auf die Thrombocytenaggregation, Relaxation der Gefäßwand, besonders der Coronararterien, sowie blutdrucksenkende Eigenschaften. Sie können daher als Arzneimittel angewandt werden.

6-keto-Prostaglandine der F-Reihe und Verfahren zu ihrer Herstellung sind bereits aus Ang. Ch. (Intern. Ed.) Vol. 17 (No. 5), S. 293—378 (1978) bekannt.

In den Patentschriften FR—A—2 376 134 und BE—A—870 531 sowie un Unlisted Drugs, Bd. 31, Nr. 4, S. 50 (Apr. 1979) sind 6-keto-$PGE_1$-Verbindungen ahnlich der Formel I der vorliegenden Anmeldung, worin $R^2$ u.a. einen alkylrest darstellt, beschrieben. Im Vergleich zu diesen Verbundungen zeigen die erfindungsgemäßen Verbindungen jedoch eine stärkere Blutplättchenaggregationshemmende Wirkung.

Die Anwendung der Verbindungen der Formel I als Blutdrucksenker erfolgt im Tagesdosisbereich von 0,001 mg — 0,1 mg/kg vorzugsweise 0,005 mg — 0,1 mg/kg bei i.V. Application bzw. im Tagesdosisbereich von 0,001 mg — 1 mg/kg, vorzugsweise 0,05 — 1 mg/kg bei oraler Applikation. Zur Relaxation der Gefäßwand, besonders der Coronararterien sowie zur Hemmung der Thrombocytenaggregation sind teilweise auch niedrigere Dosierungen wie oben angegeben wirksam.

Die erfindungsgemäßen Verbindungen der Formel I können als freie Säuren, oder in Form ihrer physiologisch unbedenklichen anorganischen oder organischen Salze oder als Ester zur Anwendung kommen. Säuren und Salze bzw. Ester können in Form ihrer wäßrigen Lösungen oder suspensionen oder auch gelöst oder suspendiert in pharmakologisch unbedenklichen organischen Lösungsmitteln wie ein- oder mehrwertigen Alkoholen wie z.B. Äthanol, Äthylenglykol oder Glycerin, Ölen wie z.B. Sonnenblumenöl oder Lebertran, Äthern wie z.B. Diäthylenglykoldimethyläther oder auch Polyäthern wie z.B. polyäthylenglykol der auch in Gegenwart anderer pharmakologisch unbedenklicher Polymerträger wie z.B. Polyvinylpyrrolidon zur Anwendung gelangen.

Als Zubereitungen können die üblichen galenischen Infusions- oder Injektionslösungen und Tabletten, sowie örtlich anwendbare zubereitungen wie Cremes, Emulsionen, suppositorien oder Aerosole in Frage kommen.

Eine weitere anwendung der neuen Verbindungen liegt in der Kombination mit anderen Wirkstoffen. Neben anderen geeigneten Substanzen gehören dazu vor allem:

Kreislaufmittel im weitesten Sinne, wie z.B. Herzglycoside wie Digitoxin, Sympathomimetica wie Suprifen, -Sympatholytica wie Inderal, Coronardilatatoren wie Chromonar oder Prenylamin, blutdrucksenkende Stoffe wie Reserpin oder Ionidin, Antiarrhythmica, durchblutungsfördernde Stoffe, Antikoagulatien oder Fibrinolytica, Diuretica wie. z.B. Furosemid, Lipidsenker, Prostaglandine oder Prostaglandinantagonisten oder Prostaglandin-Biosynthesehemmer, wie z.B. nichtsteroidale Antiphlogistika, Thomboxan-Synthetasehemmer, Psychoharmaka sowie Vitamine.

Die Verbindungen der Formel II, III, IV and V sind neue wertvolle Zwischenprodukte für die Herstellung von Verbindungen der Formel I.

**Patentansprüche für die Vertragsstaaten: BE CH DE FR GB IT LI NL SE**

1. Verbindungen der Formel I

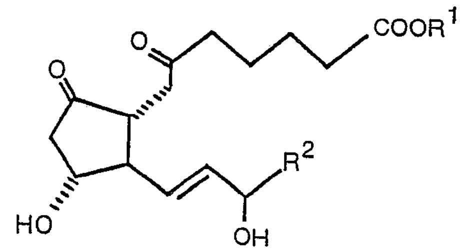

I

in welcher bedeuten:

$R^1$ Wasserstoff oder einen geradkettigen oder verzweigten Alkylrest mit bis zu 8 Kohlenstoffatomen oder einen geradkettigen oder verzweigten ungesättigten aliphatischen Kohlenwasserstoffrest mit 3 bis 6 Kohlenstoffatomen oder einen cycloaliphatischen Kohlenwasserstoffrest mit 3 bis 7 Kohlenstoffatomen oder einen araliphatischen Kohlenwasserstoffrest mit 7 bis 9 Kohlenstoffatomen, oder ein physiologisch verträgliches Metall-, $NH_4$— oder Ammoniumion, das sich von einem primären, sekundären oder tertiären Amin ableitet, oder ein Tetraalkylammoniumion,

$R^2$ einen geradkettigen oder verzweigten Alkylrest mit bis zu 8 Kohlenstoffatomen, der substituiert ist mit $\alpha$- oder $\beta$-Furyl.

2. Verfahren zur Herstellung von Verbindungen der Formel I, dadurch gekennzeichnet, daß man in einer Verbindung der Formel V

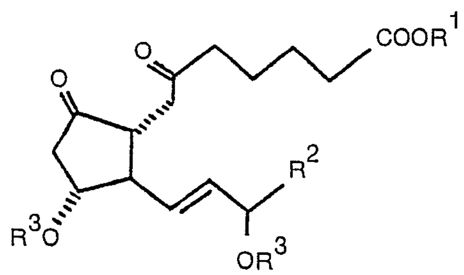

V

worin $R^1$ und $R^2$ die zur Formel I angegebene Bedeutung haben und $R^3$ eine leicht abspaltbare Schutzgruppe bedeuten, die Schutzgruppen $R^3$ unter neutralen oder sauren Bedingungen abspaltet.

3. Verfahren zur Herstellung von Verbindungen der Formel I, dadurch gekennzeichnet, daß man

a) aus einer Verbindung der Formel II

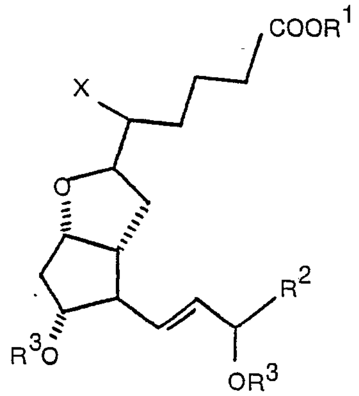

II

worin $R^1$ und $R^2$ die zur Formel I angegebene Bedeutung haben, $R^3$ eine leicht abspaltbare Schutzgruppe und X ein Halogenatom bedeuten, mit Hilfe einer Base HX abspaltet wobei eine Verbindung der Formel III

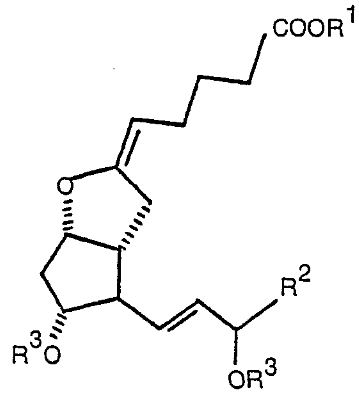

III

erhalten wird, worin $R^1$ und $R^2$ die zur Formel I angegebene Bedeutung haben und $R^3$ eine leich abspaltbare Schutzgruppe bedeutet,

b) eine Verbindung der Formel III sauer hydrolysiert zu einer Verbindung der Formel IV,

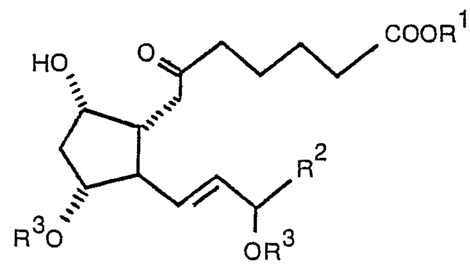

IV

worin $R^1$ und $R^2$ die zur Formel I angegebene Bedeutung haben und $R^3$ eine leicht abspaltbare Schutzgruppe bedeutet,

c) eine Verbindung der Formel IV durch Oxydation überführt in eine Verbindung der Formel V

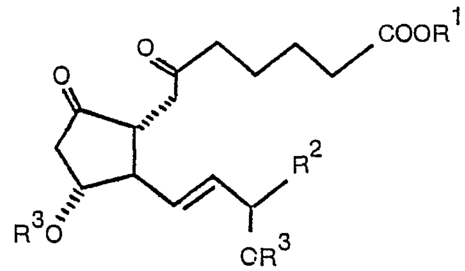

V

worin $R^1$ und $R^2$ die zur Formel I angegebene Bedeutung haben und $R^3$ eine leicht abspaltbare Schutzgruppe bedeutet,

d) in einer Verbindung der Formel V, die Schutzgruppen $R^3$ unter neutralen oder sauren Bedingungen abspaltet, wobei eine Verbindung der Formel I gebildet wird,

e) gegebenenfalls eine Verbindung der Formel I, in der $R^1$ nicht Wasserstoff oder ein Kation darstellt, durch saure oder alkalische Hydrolyse überführt in eine Verbindung der Formel I, in der $R^1$ Wasserstoff oder ein physiologisch verträgliches Kation und $R^2$ die in Formel I angegebene Bedeutung hat,

f) gegebenenfalls in einer Verbindung der Formel I, in der $R^1$ ein physiologisch verträgliches Metall-, $NH_4$- oder Ammoniumion, das sich von einem primären, sekundären oder tertiären Amin ableitet oder ein Tetraalkylammoniumion bedeutet, dieses Kation gegen ein anderes austauscht.

4. Verbindungen der Formel IV

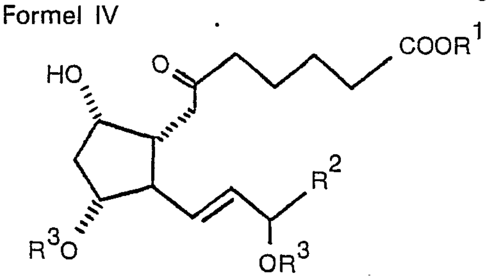

IV

worin $R^1$ und $R^2$ die zur Formel I angegebene Bedeutung haben und $R^3$ eine leicht abspaltbare Schutzgruppe bedeutet.

5. Verbindungen der Formel V

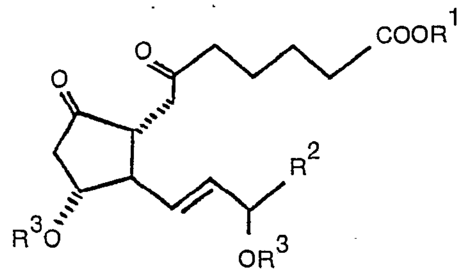

V

worin $R^1$ und $R^2$ die zur Formel I angegebene Bedeutung haben und $R^3$ eine leicht abspaltbare Schutzgruppe bedeutet.

6. Verfahren zur Herstellung von pharmazeutischen Präparaten zur Behandlung von Thrombosen und/oder Infarkten, sowie zur Behandlung des Bluthochdrucks, dadurch gekennzeichnet, daß man eine Verbindung der Formel I in Anspruch 1, gegebenenfalls mit pharmazeutisch üblichen Trägerstoffen und/oder Stabilisatoren in eine therapeutisch geeignete Anwendungsform bringt.

7. Pharmazeutische Präparate zur Behandlung von Thrombosen und/oder Infarkten, sowie zur Behandlung des Bluthochdrucks, gekennzeichnet durch einen Gehalt an einer Verbindung der Formel I in Anspruch 1.

8. Eine Verbindung der Formel I gemäß Anspruch 1 zur Verwendung als Arzneimittel.

**Patentansprüche für den Vertragsstaat: AT**

1. Verfahren zur Herstellung von Verbindungen der Formel I

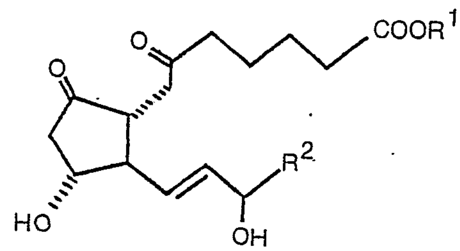

I

in welcher bedeuten:

$R^1$ Wasserstoff oder einen geradkettigen oder verzweigten Alkylrest mit bis zu 8 Kohlenstoffatomen oder einen geradkettigen oder verzweigten ungesättigten aliphatischen Kohlenwasserstoffrest mit 3 bis 6 Kohlenstoffatomen oder einen cycloaliphatischen Kohlenwasserstoffrest mit 3 bis 7 Kohlenstoffatomen oder einen araliphatischen Kohlenwasserstoffrest mit 7 bis 9 Kohlenstoffatomen, oder ein physiologisch verträgliches Metall-, $NH_4$— oder Ammoniumion, das sich von einem primären, sekundären oder tertiären Amin ableitet, oder ein Tetraalkylammoniumion,

$R^2$ einen geradkettigen oder verzweigten Alkylrest mit bis zu 8 Kohlenstoffatomen, der substituiert ist mit $\alpha$- oder $\beta$-Furyl, dadurch gekennzeichnet, daß man eine Verbindung der Formel IV

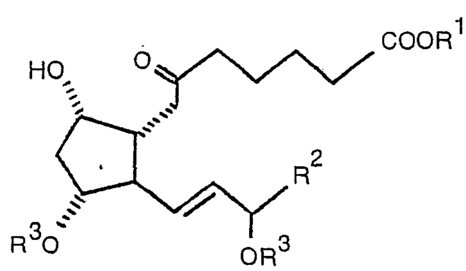

IV

worin $R^1$ und $R^2$ die zur Formel I angegebene Bedeutung haben und $R^3$ eine leicht absaltbare Schutzgruppe bedeutet,

a) durch Oxydation überführt in eine Verbindung der Formel V

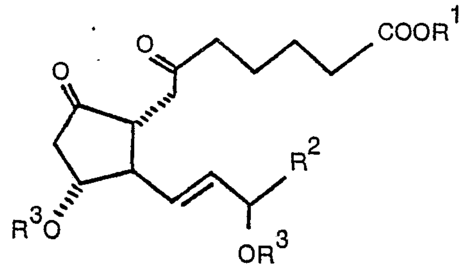

V

worin $R^1$ und $R^2$ die zur Formel I angegebene Bedeutung haben und $R^3$ eine leicht abspaltbare Schutzgruppe bedeutet,

b) in einer Verbindung der Formel V die Schutzgruppen $R^3$ unter neutralen oder sauren Bedingungen abspaltet, wobei eine Verbindung der Formel I gebildet wird,

c) gegebenenfalls eine Verbindung der Formel I, in der $R^1$ nicht Wasserstoff oder ein Kation darstellt, durch saure oder alkalische Hydrolyse überführt in eine Verbindung der Formel I, in der $R^1$ Wasserstoff oder ein physiologisch verträgliches Kation und $R^2$ die in Formel I angegebene Bedeutung hat,

d) gegebenenfalls in einer Verbindung der Formel I, in der $R^1$ ein physiologisch verträgliches Metall, $NH_4$- oder Ammonium, das sich von einem primären, sekundären oder tertiären Amin ableitet, dieses Kation gegen ein anderes austauscht.

2. Verfahren zur Herstellung von pharmazeutischen Präparaten zur Behandlung von Thrombosen und/oder Infarkten, sowie zur Behandlung des Bluthochdrucks, dadurch gekennzeichnet, daß man eine Verbindung der Formel I in Anspruch 1, gegebenenfalls mit pharmazeutisch üblichen Trägerstoffen und/oder Stabilisatoren in eine therapeutisch geeignete Anwendungsform bringt.

**Revendications pour les Etats contractants: BE CH DE FR GB IT LI NL SE**

1. Composés de formule I

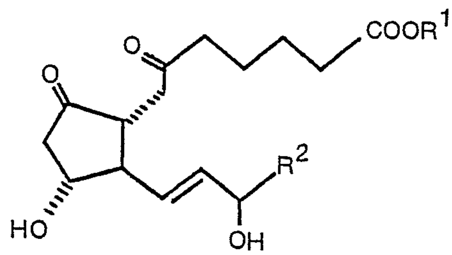

I

dans laquelle;

$R^1$ représente l'hydrogène ou un groupe alcoyle à chaîne droite ou ramifiée ayant jusqu'à 8 atomes de carbone ou un groupe hydrocarbure aliphatique à chaîne droite ou ramifiée ayant de 3 à 6 atomes de carbone ou un groupe hydrocarbure cycloaliphatique ayant de 3 à 7 atomes de carbone ou un groupe hydrocarbure araliphatique ayant de 7 à 9 atomes de carbone, ou un ion métallique physiologiquement acceptable, $NH_4$ ou ammonium qui dérive d'une amine primaire, secondaire ou tertiaire, ou un ion tétraalcoylammonium,

$R^2$ représente un groupe alcoyle à chaîne droite ou ramifiée ayant jusqu'à 8 atomes de carbone, qui est substitué par un groupe $\alpha$ ou $\beta$-furyle.

2. Procédé de préparation de composés de formule I, caractérisée en ce que, dans un composé de formule V

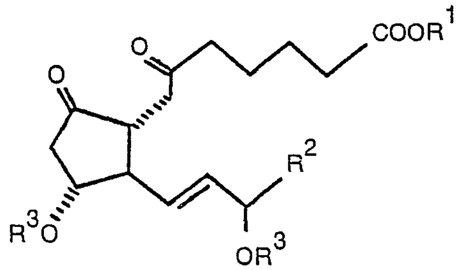

V

dans laquelle $R^1$ et $R^2$ ont les significations indiquées pour la formule I et $R^3$ représente un groupe protecteur facilement éliminable, on élimine les groupes protecteurs $R^3$ dans des conditions neutres ou acides.

3. Procédé de préparation de composés de formule I, caractérisé en ce que:

a) à partir d'un composé de formule II

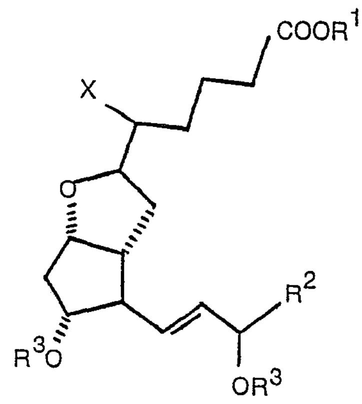

II

dans laquelle $R^1$ et $R^2$ ont les significations indiquées pour la formule I, $R^3$ représente un groupe protecteur facilement éliminable et X représente un atome d'halogène, on élimine HX à l'aide d'une base et on obtient un composé de formule III

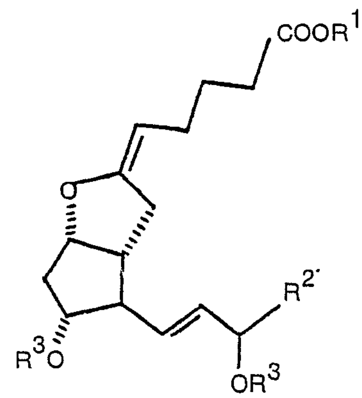

III

dans laquelle $R^1$ et $R^2$ ont les significations indiquées pour la formule I et $R^3$ représente un groupe protecteur facilement éliminable,

b) on hydrolyse par voie acide un composé de formule III en un composé de formule IV.

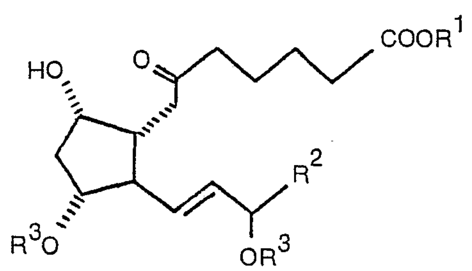

IV

dans laquelle $R^1$ et $R^2$ ont les significations indiquées pour la formule I et $R^3$ représente un groupe protecteur facilement éliminable,

c) on convertit un composé de formule IV par oxydation en un composé de formule V

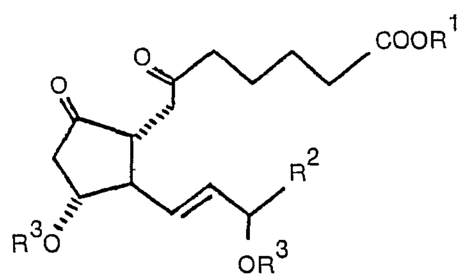

V

dans laquelle $R^1$ et $R^2$ ont les significations indiquées pour la formule I et $R^3$ représente un groupe protecteur facilement éliminable;

d) dans un composé de formule V, on élimine les groupes protecteurs $R^3$ dans des conditions neutres ou acides, et on obtient un composé de formule I,

e) éventuellement on convertit un composé de formule I, dans lequel $R^1$ ne représente pas l'hydrogène, ni un cation, par hydrolyse acide ou alcaline en un composé de formule I, dans lequel $R^1$ représente l'hydrogène ou un cation physiologiquement acceptable et $R^2$ a la signification indiquée pour la formule I,

f) éventuellement dans un composé de formule I, dans lequel $R^1$ représente un ion métallique physiologiquement acceptable, $NH_4$ ou ammonium qui dérive d'une amine primaire, secondaire ou tertiaire, ou un ion tétraalcoylammonium, on échange ce cation contre un autre.

4. Composés de formule IV

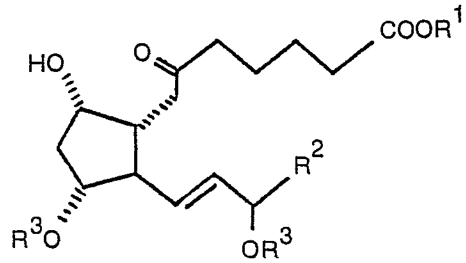

IV

dans laquelle $R^1$ et $R^2$ ont les significations indiquées pour la formule I et $R^3$ représente un groupe protecteur facilement éliminable.

5. Composés de formule V

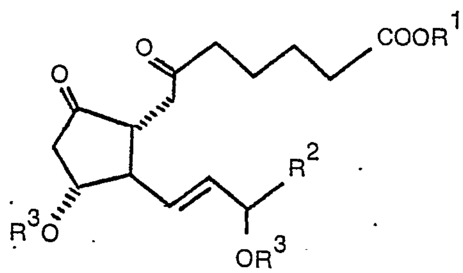

V

dans laquelle $R^1$ et $R^2$ ont les significations indiquées pour la formule I et $R^3$ représente un groupe protecteur facilement éliminable.

6. Procédé de préparation de compositions pharmaceutiques pour le traitement de thromboses et/ou d'infarctus, ainsi que pour le traitement de l'hypertension sanguine, caractérisé en ce qu'on met un composé de formule I selon la revendication 1, sous une forme thérapeutiquement appropriée, éventuellement avec des véhicules et/ou des stabilisants pharmaceutiquement usuels.

7. Compositions pharmaceutiques pour le traitement des thromboses et/ou des infarctus, ainsi que pour le traitement de l'hypertension sanguine, caractérisé par une teneur en un composé de formule I selon la revendication 1.

8. Composé de formule I selon la revendication 1 pour application thérapeutique.

**Revendications pour l'Etat contractant: AT**

1. Procédé pour la préparation de composés de formule I

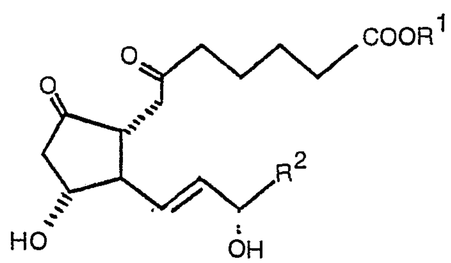

I

dans laquelle;

$R^1$ représente l'hydrogène ou un groupe alcoyle à chaîne droite ou ramifiée, ayant jusqu'à 8 atomes de carbone, ou un groupe hydrocarbure aliphatique insaturé à chaîne droite ou

ramifiée, ayant de 3 à 6 atomes de carbone, ou un groupe hydrocarbure cycloaliphatique ayant de 3 à 7 atomes de carbone ou un groupe hydrocarbure araliphatique ayant de 7 à 9 atomes de carbone, ou un ion métallique physiologiquement acceptable, $NH_4$ ou ammonium qui dérive d'une amine primaire, secondaire ou tertiaire, ou un ion tétraalcoylammonium,

$R^2$ représente un groupe alcoyle à chaîne droite ou ramifiée, ayant jusqu'à 8 atomes de carbone, qui est substitué par un groupe $\alpha$ ou $\beta$-furyle, caractérisé en ce que

a) on convertit par oxydation en un composé de formule IV

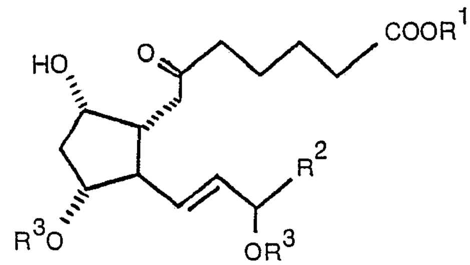

IV

dans laquelle $R^1$ et $R^2$ ont les significations indiquées pour la formule I et $R^3$ représente un groupe protecteur facilement éliminable en un composé de formule V

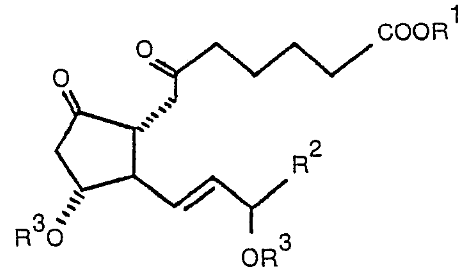

V

dans laquelle $R^1$ et $R^2$ ont les significations indiquées pour la formule I et $R^3$ représente un groupe protecteur facilement éliminable.

b) dans un composé de formule V, on élimine les groupes protecteurs $R^3$ dans des conditions neutres ou acides, et on obtient un composé de formule I,

c) éventuellement on convertit un composé de formule I, dans lequel $R^1$ ne représente pas l'hydrogène, ni un cation, par hydrolyse acide ou alcaline, en un composé de formule I, dans lequel $R^1$ représente l'hydrogène ou un cation physiologiquement acceptable et $R^2$ a la signification indiquée pour la formule I,

d) éventuellement, dans un composé de formule I, dans lequel $R^1$ représente un ion métallique physiologiquement acceptable, $NH_4$ ou ammonium qui dérive d'une amine primaire, secondaire ou tertiaire, on échange ce cation contre un autre.

2 Procédé de préparation de compositions pharmaceutiques pour le traitement des thromboses et/ou des infarctus, ainsi que pour le traitement de l'hypertension, caractérisé en ce qu'on met un composé de formule I selon la revendication 1, sous une forme thérapeutiquement appropriée éventuellement avec des véhicules et/ou des stabilisants pharmaceutiquement usuels.

**Claims for the Contracting States: BE CH DE FR GB IT LI NL SE**

1. Compounds of the formula I

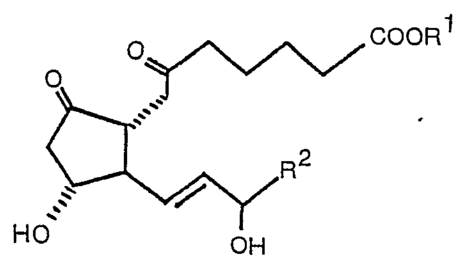

I

in which $R^1$ denotes hydrogen or a straight-chain or branched alkyl radical with up to 8 carbon atoms or a straight-chain or branched unsaturated aliphatic hydrocarbon radical with 3 to 6 carbon atoms or a cycloaliphatic hydrocarbon radical with 3 to 7 carbon atoms or an araliphatic hydrocarbon radical with 7 to 9 carbon atoms or a physiologically acceptable metal ion, $NH_4$ ion or ammonium ion derived from a

primary, secondary or tertiary amine, or a tetraalkylammonium ion and $R^2$ denotes a straight-chain or branched alkyl radical with up to 8 carbon atoms, which is substituted with an $\alpha$- or $\beta$-furyl radical.

2. Process for the manufacture of compounds of the formula I, which comprises detaching the protective groups $R^3$, under neutral or acid conditions, from a compound of the formula V

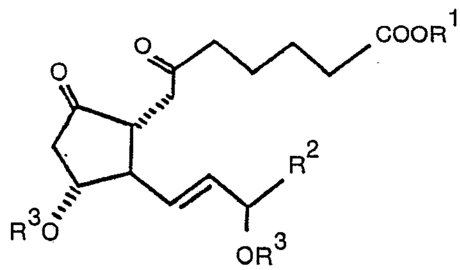

V

in which $R^1$ and $R^2$ have the meaning indicated under formula I and $R^3$ denotes an easily detachable protective group.

3. Process for the manufacture of compounds of the formula I, which comprises a) eliminating HX, with the aid of a base, from a compound of the formula II

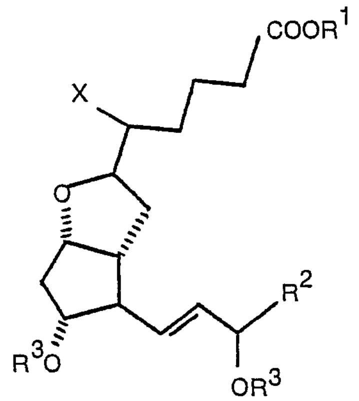

II

in which $R^1$ and $R^2$ have the meaning indicated under formula I, $R^3$ denotes an easily detachable protective group and X denotes a halogen atom, a compound of the formula III

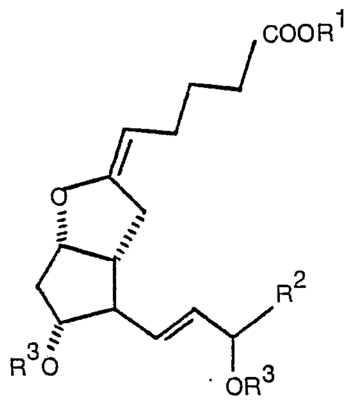

III

being obtained, in which $R^1$ and $R^2$ have the meaning indicated under formula I and $R^3$ denotes an easily detachable protective group, b) subjecting a compound of the formula III to acid hydrolysis to give a compound of the formula IV

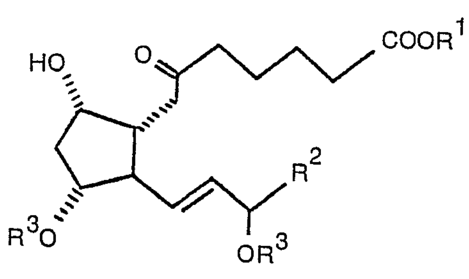

IV

in which $R^1$ and $R^2$ have the meaning indicated under formula I and $R^3$ denotes an easily detachable protective group, c) converting a compound of the formula IV by oxidation to a compound of the formula V

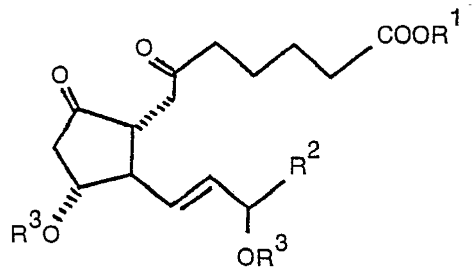

V

in which $R^1$ and $R^2$ have the meaning indicated under formula I and $R^3$ denotes an easily detachable protective group, d) detaching the protective groups $R^3$ from a compound of the formula V under neutral or acid conditions, a compound of the formula I being formed, and e) if desired, converting a compound of the formula I in which $R^1$ does not represent hydrogen or a cation by acid or alkaline hydrolysis to a compound of the formula I in which $R^1$ denotes hydrogen or a physiologically acceptable cation and $R^2$ has the meaning indicated in formula I, and f) is desired, in a compound of the formula I in which $R^1$ denotes a physiologically acceptable metal ion, $NH_4$ ion or ammonium ion, derived from a primary, secondary or tertiary amine, or a tetraalkylammonium ion, replacing this cation by another cation.

4. Compounds of the formula IV

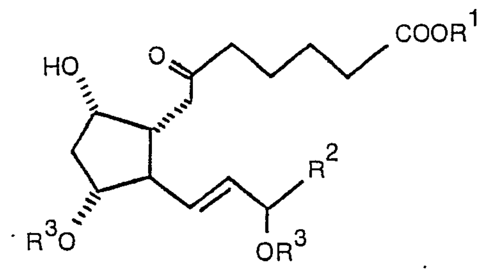

IV

in which $R^1$ and $R^2$ have the meaning indicated under formula I and $R^3$ denotes an easily detachable protective group.

5. Compounds of the formula V

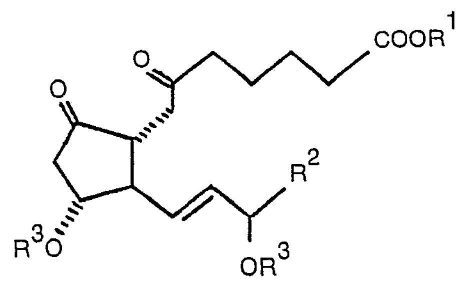

V

in which $R^1$ and $R^2$ have the meaning indicated under formula I and $R^3$ denotes an easily detachable protective group.

6. Process for the manufacture of pharmaceutical preparations for the treatment of thromboses and/or infarcts, and also for the treatment of hypertension, wherein a compound of the formula I in Claim 1, optionally together with pharmaceutically customary excipients and/or stabilizers, is brought into a therapeutically suitable use form.

7. Pharmaceutical preparations for the treatment of thromboses and/or infarcts and also for the treatment of hypertension, which contain a compound of the formula I in claim 1.

8. A compound of the formula I as defined in claim 1 for use as a medicament.

**Claims for the Contracting State: AT**

1. Process for the manufacture of compounds of the formula I

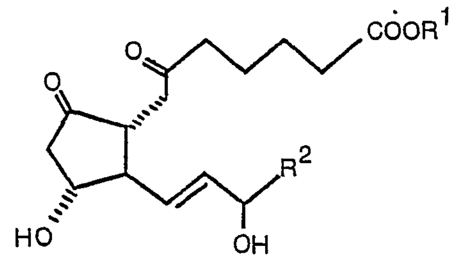

I

in which $R^1$ denotes hydrogen or a straight-chain or branched alkyl radical with up to 8 carbon atoms or a straight-chain or branched unsaturated aliphatic hydrocarbon radical with 3 to 6 carbon atoms or a cycloaliphatic hydrocarbon radical with 3 to 7 carbon atoms or an araliphatic hydrocarbon radical with 7 to 9 carbon atoms or a physiologically acceptable metal ion, $NH_4$ ion or ammonium ion derived from a primary, secondary or tertiary amine, or a tetraalkylammonium ion and $R^2$ denotes a straight-chain or branched alkyl radical with up to 8 carbon atoms, which is substituted with an $\alpha$- or $\beta$-furyl radical, which comprises a) converting a compound of the formula IV

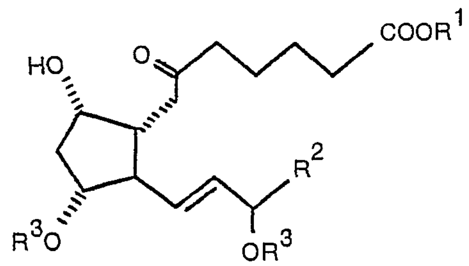

IV

in which $R^1$ and $R^2$ have the meaning indicated under formula I and $R^3$ denotes an easily detachable protective by oxidation to a compound of the formula V

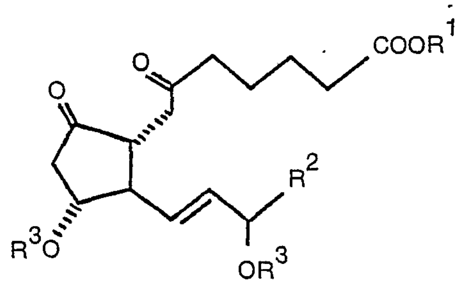

V

in which $R^1$ and $R^2$ have the meaning indicated under formula I and $R^3$ denotes an easily detachable protective group, b) detaching the protective groups $R^3$ from a compound of the formula V under neutral or acid conditions, a compound of the formula I being formed, and c) if desired, converting a compound of the formula I in which $R^1$ does not represent hydrogen or a cation by acid or alkaline hydrolysis to a compound of the formula I in which $R^1$ denotes hydrogen or a physiologically acceptable cation and $R^2$ has the meaning indicated in formula I, and d) if desired, in a compound of the formula I in which $R^1$ denotes a physiologically acceptable metal ion, $NH_4$ ion or ammonium ion, derived from a primary, secondary or tertiary amine, replacing this cation by another cation.

2. Process for the manufacture of pharmaceutical preparations for the treatment of thromboses and/or infarcts, and also for the treatment of hypertension, wherein a compound of the formula I in Claim 1, optionally together with pharmaceutically customary excipients and/or stabilizers, is brought into a therapeutically suitable use form.